# EUROPEAN PATENT SPECIFICATION

(11) **EP 0 871 487 B1**
(45) Date of publication and mention of the grant of the patent: **27.05.2009**
(21) Application number: 96936216.9
(22) Date of filing: 03.10.1996
(51) Int. Cl.: A61K 45/06, A61K 39/12, A61K 39/02, A61K 39/00, A01N 1/02, C12N 5/00

(54) **DENDRITIC CELL STIMULATORY FACTOR**
STIMULATIONSFAKTOR FÜR DENDRITEN
FACTEUR DE STIMULATION DE CELLULES DENDRITIQUES

(30) Priority: 04.10.1995 US 539142
(43) Date of publication of application: 21.10.1998
(73) Proprietor: Immunex Corporation, Thousand Oaks, CA 91320-1799 (US)
(72) Inventor: BRASEL, Kenneth, Seattle, WA 98117 (US); LYMAN, Stewart, D., Seattle, WA 98103 (US); MARASKOVSKY, Eugene, Seattle, WA 98103 (US); McKENNA, Hilary R., Seattle, WA 98103 (US); LYNCH, David, H., Bainbridge Island, WA 98110 (US)
(74) Representative: Cornish, Kristina Victoria Joy
(86) International application number: PCT/US1996/015990
(87) International publication number: WO 1997/012633

(56) References cited:
- EP-A- 0 627 487
- WO-A-95/00632
- BROXMEYER HAL E ET AL: "Flt3 ligand stimulates/costimulates the growth of myeloid stem/progenitor cells" EXPERIMENTAL HEMATOLOGY (CHARLOTTESVILLE), vol. 23, no. 10, 1995, pages 1121-1129, XP008032698 ISSN: 0301-472X
- MARASKOVSKY E ET AL: "The effect of FLT3 ligand and/or c-kit ligand on the generation of dendritic cells from human CD34+ bone marrow" BLOOD, vol. 86, no. 10 SUPPL. 1, 1995, page 420A, XP008032700 & 37TH ANNUAL MEETING OF THE AMERICAN SOCIETY OF HEMATOLOGY; SEATTLE, WASHINGTON, USA; DECEMBER 1-5, 1995 ISSN: 0006-4971
- BLOOD, 15 May 1995, Vol. 85, No. 10, HUDAK et al., "FLT3/FLK2 Ligand Promotes the Growth of Murine Stem Cells and the Expansion of Colony-Forming Cells and Spleen Colony-Forming Units", pages 2747-2755. XP002980731
- NATURE, 14 April 1994, Vol. 368, HANNUM et al., "Ligand for FLT3/FLK2 Receptor Tyrosine Kinase Regulates Growth of Haematopoietic Stem Cells and is Encoded by Variant RNAs", pages 643-648. XP002057419
- BLOOD, 15 February 1995, Vol. 85, MUENCH et al., "FLK-2/FLT-3 Ligand Regulates the Growth of Early Myeloid Progenitors Isolated From Human Fetal Liver", pages 963-972. XP002980732
- ANNU. REV. IMMUNOL., 1991, Vol. 9, STEINMAN, "The Dendritic Cell System and its Role in Immunogenicity", pages 271-296. XP000856705
- IMMUNOLOGY, 1991, Vol. 74, MACATONIA et al., "Primary Proliferative and Cytotoxic T-Cell Responses to HIV Induced in Vitro by Human Dendritic Cells", pages 399-406. XP002910826
- IMMUNOLOGY, 1992, Vol. 76, PANCHOLI et al., "Dendritic Cells Efficiently Immunoselect Mycobacterial-Reactive T Cells in Human Blood, Including Clonable Antigen-Reactive Precursors", pages 217-224. XP002082271
- J. EXP. MED., August 1990, Vol. 172, INABA et al., "Dendritic Cells Pulsed With Protein Antigens in Vitro Can Prime Antigen-Specific, MHC-Restricted T Cells in Situ", pages 631-640. XP000572923
- INTERN. REV. IMMUNOL., 1990, Vol. 6, BUJDOSO et al., "Afferent Lymph Dendritic Cells: a Model for Antigen Capture and Presentation in Vivo", pages 177-186. XP002980745
- PEDIATRIC PATHOLOGY, 1993, Vol. 13, JAFFE, "Review of Human Dendritic Cells: Isolation and Culture From Precursors", pages 821-837. XP000651306
- BLOOD CELLS, 1994, Vol. 20, BERNSTEIN et al., "Isolation of Human Hematopoietic Stem Cells", pages 15-24. XP002980746
- J. EXP. MED., October 1995, Vol. 182, YOUNG et al., "Identification of Dentritic Cell Colony-Forming Units Among Normal Human CD34+ Bone Marrow Progenitors that are Expanded by C-Kit-Ligand and Yield Pure Dendritic Cell Colonies in the Presence of Granulocyte/Macrophage Colony-Stimulating Factor and XP002980733
- J. EXP. MED., August 1993, Vol. 178, INABA et al., "Dendritic Cell Progenitors Phagocytose Particulates, Including Bacillus Calmette-Guerin Organisms and Sensitize Mice to Mycobacterial Antigens in Vivo", pages 479-488. XP002937826

## Description

### FIELD OF THE INVENTION

The present invention relates to uses of a dendritic cell stimulatory factor.

### BACKGROUND OF THE INVENTION

The objective of vaccination is to provide effective immunity by establishing adequate levels of antibody and a primed population of cells that can rapidly expand on renewed contact with antigen. The first contact with antigen during vaccination must not be injurious to the recipient and thus usually consists of pathogenically-deficient antigen.

A frequent difficulty with active immunization protocols is that the vaccine antigen does not possess sufficient immunogenicity to promote a strong immune response, and therefore a sufficient level of protection against subsequent challenge by the same antigen. In addition, certain antigens may elicit only weak cell-mediated or antibody response. For many antigens, both a strong humoral response and a strong cell-mediated response is desirable.

For decades, researchers have experimented with diverse compounds to increase the immunogenicity of vaccines. Immunopotentiators, also known as adjuvants, of vaccines are compositions of matter that facilitate a strong immune response to a vaccine. In addition, the relatively weak immunogenicity of certain novel recombinant antigens has required adjuvants to be more potent. Vaccine adjuvants have different modes of action, affecting the immune response both quantiatively and qualitatively. Such modes of action can be by mobilizing T cells, acting as depots and altering lymphocyte circulation so that these cells remain localized in draining lymph nodes. They may also serve to focus antigen at the site of immunization, thereby allowing antigen specific T cells and B cells to interact more efficiently with antigen-presenting cells. They may also stimulate proliferation and differentiation of T cells and have effects on B cells, such as enhancing the production of different Ig isotypes. Further, adjuvants may stimulate and affect the behaviour of antigen-presenting cells, particularly macrophages, rendering them more effective for presenting antigen to T cells and B cells.

Dendritic cells are a rare and heterogeneous cell population with distinctive morphology and a widespread tissue distribution. A discussion of the dendritic cell system and its role in immunogenicity is provided by Steinman, R.M., Annu. Rev. Immunol., 9:271-296 (1991). Dendritic cells display an unusual cell surface and can be characterized by the presence of the cell surface markers CD1a⁺, CD4⁺, CD14⁻ CD86⁺, CD11c⁺, DEC-205⁺, CD14⁺ or HLA-DR⁺. Dendritic cells have a high capacity for sensitizing MHC-restricted T cells and provide an effective pathway for presenting antigens to T cells in situ, both self-antigens during T cell development and foreign antigens during immunity. Thus, there is growing, interest in using dendritic cells ex vivo as tumor or infectious disease vaccine adjuvants. See, for example, Romani, et al., J. Exp. Med., 180:83 (1994). The use of dendritic cells as immunostimulatory agents has been limited due to the low frequency of dendritic cells in peripheral blood, the limited accessibility to lymphoid organs and the dendritic cells' terminal state of differentiation. Dendritic cells originate from CD34⁺ bone marrow progenitors, and the proliferation and maturation of dendritic cells can be enhanced by the cytokines GM-CSF (sargramostim, Leukine^{®}, Immunex Corporation, Seattle, Washington), TNF-α, c-kit ligand (also known as stem cell factor (SCF), steel factor (SF), or mast cell growth factor (MGF)) and interleukin-4. Therefore, an agent that stimulated the generation of large numbers of functionally mature dendritic cells in vivo or in vitro would be of wide importance.

### SUMMARY OF THE INVENTION

Flt3-ligand ("flt3-ligand" or "flt3-L") is known to affect hematopoietic stem and progenitor cells. It was surprisingly found that flt3-ligand can also potently stimulate the generation of dendritic cells from CD34⁺ bone marrow progenitors and stem cells. The present invention is as set out in the claims.

The invention provides for flt3-ligand for use as a vaccine adjuvant for increasing the number of dendritic cells. By increasing the quantity of the patient's dendritic cells, such cells may themselves be used to present antigen to T cells. For example, the antigen may be one that already exists within the patient, such as a tumor antigen, or a bacterial or viral antigen. Flt3-ligand may be used, therefore, to increase the numbers of dendritic in vivo to boost a patient's immune response against existing antigens. Alternatively, flt3-ligand may be administered prior to, concurrently with or subsequent to administration of an antigen to a patient for immunization purposes. Thus, as a vaccine adjuvant, flt3-ligand can generate large quantities of dendritic cells and other intermediate cells in vivo to more effectively present the antigen. The overall response is a stronger and improved immune response and more effective immunization to the antigen.

The invention further comprises flt3-ligand for use in increasing dendritic cells in a host, a donor or both undergoing a transplantation procedure, whereby the increased number of dendritic cells inhibit graft versus host and host versus graft disease.

The invention also comprises flt3-ligand for use in treating fibrosarcoma.

The invention can further comprise the use of an effective amount of a cytokine in sequential or concurrent combination with flt3-ligand. Such cytokines include, but are not limited to, interleukins ("ILs") IL-3 and IL-4, a colony stimulating factor ("CSF") selected from the group consisting of granulocyte macrophage colony stimulating factor ("GM-CSF") or GM-CSF/IL-3 fusions, or other cytokines such as TNFa or c-kit ligand.

The invention further includes uses of flt3-ligand in combination with a cytokine from the group listed above in a cell expansion media for generating dendritic cells in *vitro.*

### DETAILED DESCRIPTION OF THE INVENTION

The invention is directed to the use of flt3-ligand to generate large numbers of dendritic cells for the uses defined in the claims. The large numbers of dendritic cells are not naturally found in vivo and can be generated by administering flt3-ligand. Such enhancement in overall cell number can augment the immune response to antigen in the host and therefore flt3-ligand can be used as a vaccine adjuvant. The invention is also directed to use of flt3-ligand and any one of cytokines IL-3, IL-4, GM-CSF, TNF, C-Kit ligand and GM-CSF/IL-3 fusion proteins in a cell expansion media for generating dendritic cells in vitro.

As used herein, the term "flt3-ligand" refers to a genus of polypeptides that are described in United States Patent No. 5,554,512, EP 0627487 A2 and in WO 94/28391. A human flt3-ligand cDNA was deposited with the American Type Culture Collection, Rockville, Maryland, USA (ATCC) on August 6, 1993 and assigned accession number ATCC 69382. The deposit was made under the terms of the Budapest Treaty. Flt3-ligand can be made according to the methods described in the documents cited above.

The term "IL-3" refers to a genus of interleukin-3 polypeptides as described in U.S. Patent No. 5,108,910. Such polypeptides include analogs that have amino acid sequences that are substantially similar to the native human interleukin-3 amino acid sequences disclosed, for example, in EP publ. Nos. 275,598 and 282,185. The term "IL-3" also includes analogs and alleles of IL-3 molecules that exhibit at least some of the biological activity in common with native human IL-3. Exemplary analogs of IL-3 are disclosed in EP Publ. No. 282,185. Other forms of IL-3 include human IL-3[Pro⁸Asp¹⁵Asp⁷⁰], human IL-3[Ser⁸Asp¹⁵Asp⁷⁰] and human IL-3[Ser⁸]. A DNA sequence encoding human IL-3 protein suitable for use in the invention is publicly available from the American Type Culture Collection (ATCC) under accession number ATCC 67747. The nomenclature used herein with respect to amino acid sequences in brackets designates which amino acids differ from the native human form. For example, human IL-3[Ser⁸Asp¹⁵Asp⁷⁰] refers to a human IL-3 protein in which amino acid 8 has been changed to a serine residue, amino acid 15 has been changed to an aspartic acid residue and the amino acid 70 has been changed to an aspartic acid residue.

The term "IL-4" refers to a polypeptide as described in Mosley et al., Cell 59:335 (1989), Idzerda et al., J. Exp. Med. 171:861 (1990) and Galizzi et al., Intl. Immunol. 2:669 (1990). Such IL-4 polypeptide includes analogs that have an amino acid sequence that is substantially similar to the native human IL-4 amino acid sequences described in Mosley et al., Idzerda et al., and Galizzi et al. and which are biologically active in that they are capable of binding to a IL-4 receptor, transducing a biological signal initiated by binding IL-4 receptor, or cross-reacting with anti-IL-4 antibodies. The term "IL-4" also includes analogs of native human IL-4 molecules sufficient to retain biological activity of native human IL-4.

As used herein, "GM-CSF" refers to a genus of proteins as described in U.S. Patent Nos. 5,108,910, and 5,229,496. Such proteins include analogs that have an amino acid sequence that is substantially similar to native human GM-CSF amino acid sequences (e.g., as publicly available ATCC 53157 or ATCC 39900), and which are biologically active in that they are capable of binding to a GM-CSF receptor, transducing a biological signal initiated by binding GM-CSF receptor, or cross-reacting with anti-GM-CSF antibodies. Amino acid sequences are disclosed, for example in Anderson, et al., Proc. Natl. Acad. Sci., USA 82:6250 (1985). Commercially available GM-CSF (sargramostim, Leukine^{®}) is obtainable from Immunex Corp., Seattle, WA). The term "GM-CSF" also includes analogs of the native human GM-CSF molecules described in U.S. Patent Nos. 5,108,910, and 5,229,496 sufficient to retain biological activity of native human GM-CSF. Exemplary analogs of GM-CSF include, for example, those described in EP Publ. No. 212914 and WO 89/03881. Other analogs of GM-CSF also may be used to construct fusion proteins with IL-3. A DNA sequence encoding a particularly preferred GM-CSF protein having potential glycosylation sites removed is publicly available from the ATCC under accession numbers ATCC 67231.

The term "GM-CSF/IL-3 fusion protein" means a C-terminal to N-terminal fusion of GM-CSF and IL-3. The fusion proteins are known and are described in U.S, Patent Nos. 5,199,942, 5,108,910 and 5,073,627. A preferred fusion protein is PIXY321 as described in US Patent No. 5,199,942.

The term "c-kit ligand" also known as Mast Cell Growth Factor (MGF), Steel Factor or Stem Cell Factor (SGF), refers to a polypeptide described in EP 423,980, and that claims priority from U.S. Patent application Serial No. 589,701, filed October 1, 1990. Such c-kit ligand polypeptide includes analogs that have an amino acid sequence that is substantially similar to the native human c-kit ligand amino acid sequences described in EP 423,980 and which are biologically active in that they are capable of binding to a c-kit receptor, transducing a biological signal initiated by binding c-kit receptor, or cross-reacting with anti-c-kit ligand antibodies, The term "c-kit ligand' also includes analogs of native human c-kit ligand molecules sufficient to retain biological activity of native human c-kit ligand.

The term "adjuvant" refers to a substance that enhances, augments or potentiates the host's immune response to a vaccine antigen.

The procedure for "ex vivo expansion" of hematopoietic stem and progenitor cells is described in U.S. Patent No.5,199,942. Briefly, the term means a method comprising: (1) collecting CD34⁺ hematopoietic stem and progenitor cells from a patient from peripheral blood harvest or bone marrow explants: and (2) expanding such cells *ex vivo.* In addition to the cellular growth factors described in Patent 5,199,942, other factors such as flt3-ligand, IL-I, IL-3, c-kit ligand, can be used.

The term "immunogenicity" means relative effectiveness of an immunogen or antigen to induce an immune response.

The term "substantially similar" means a variant amino acid sequence preferably that is at least 80% identical to a native amino acid sequence, most preferably at least 90% identical. The percent identity may be determined, for examples, by comparing sequence information using the GAP computer program, version 6.0 described by Devereux et al. (Nucl. Acids Res. 12:387, 1984) and available from the University of Wisconsin Genetics Computer Group (UWGCG). The GAP program utilizes the alignment method of Needleman and Wunsch (J. Mol. Biol. 48:443, 1970), as revised by Smith and Waterman (Adv. Appl. Math 2:482, 1981). The preferred default parameters for the GAP program include: (1) a unary comparison matrix (containing a value of 1 for identities and 0 for non-identities) for nucleotides, and the weighted comparison matrix of Gribskov and Burgess, Nucl. Acids Res. 14:6745, 1986, as described by Schwartz and Dayhoff, eds., Atlas of Protein Sequence and Structure, National Biomedical Research Foundation, pp. 353-358, 1979; (2) a penalty of 3.0 for each gap and an additional 0.10 penalty for each symbol in each gap; and (3) no penalty for end gaps. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring variants are also encompassed by the invention. Examples of such variants are proteins that result from alternate mRNA splicing events or from proteolytic cleavage of the native protein, wherein the native biological property is retained.

As used herein, "vaccine" means an organism or material that contains an antigen in an innocuous form. The vaccine is designed to trigger an immunoprotective response. The vaccine may be recombinant or non-recombinant When inoculated into a non-immune host, the vaccine will provoke active immunity to the organism or material, but will not cause disease. Vaccines may take the form, for example, of a toxoid, which is defined as a toxin that has been detoxified but that still retains its major immunogenic determinants; or a killed organism, such as typhoid, cholera and poliomyelitis; or attenuated organisms, that are the live, but non-virulent, forms of pathogens, or it may be antigen encoded by such organism, or it may be a live tumor cell or an antigen present on a tumor cell.

A variety of cell selection techniques are known for identifying and separating CD34⁺ hematopoietic stem or progenitor cells from a population of cells. Methods and materials for identifying and selecting such cell types are known. For example, monoclonal antibodies can be used to bind to a marker protein or surface antigen protein found on stem or progenitor cells. Such markers or cell surface antigens for hematopoietic stem cells include CD34 and Thy-1. In one method, antibodies are fixed to a surface, for example, glass beads, and contacted with a mixture of cells suspected of containing stem cells. This permit the antibodies to bind and secure the stem cells to the glass beads. Alternatively, the antibodies can be incubated with the cell mixture and the resulting combination contacted with a surface having an affinity for the antibody-cell complex. Undesired cells and cell matter are removed providing a relatively pure population of stem cells. Stem or progenitor cells having the CD34 marker constitute only about% to 3% of the mononuclear cells in the bone marrow. The amount of CD34⁺ stem or progenitor cells in the peripheral blood is approximately 10- to 100-fold less than in bone marrow.

With regard to the particular aspects of the invention, choosing suitable stem or progenitor cell selection means will depend on the desired phenotype of the cell to be isolated. Hematopoietic stem cells are selectable by virtue of their physical characteristics, such as expressing the membrane-bound flt3 receptor, or having the following cellular markers: CD34 or Thy-1. Monoclonal antibodies that recognize any of these antigens have been described in U.S. Patent No. 4,714,680 (anti-My-10), anti-CD34 is commercially available from Becton Dickinson, Franklin Lakes, NJ), and anti-Thy-1 monoclonal antibodies can be readily generated using the methods described by Dalchau et al., J. Exp. Med. 149:576 (1979). A flt3 preceptor binding protein also may be used, such as anti-flt3 monoclonal antibodies or the flt3-ligand. The cell binding protein is brought into contact with the collected cell mixture and the combination is allowed to incubate for a period of time sufficient to permit the binding of the desired cell to the cell binding protein.

An alternative means of selecting the quiescent stem cells is to induce cell death in the dividing, more lineage-committed, cell types using an antimetabolite such as 5-fluorouracil (5-FU) or an alkylating agent such as 4-hydroxycyclophosphamide (4-HC). The non-quiescent cells are stimulated to proliferate and differentiate by the addition of growth factors that have little or no effect on the stem cells, causing the non-stem cells to proliferate and differentiate and making them more vulnerable to the cytotoxic effects of 5-FU or 4-HC. See Berardi et al., Science, 267: 104 (1995).

Isolation of the hematopoietic stem or progenitor cells can be performed by using, for example, affinity chromatography, antibody-coated magnetic beads, or antibodies fixed to a solid matrix, such is glass beads, flasks, etc. Antibodies that recognize a stem or progenitor cell surface marker can be fused or conjugated to other chemical moieties such as biotin - which can be removed with an avidin or a streptavidin moiety secured to a solid support; fluorochromes useful in fluorescence activated cell sorting (FACS), or the like. Preferably, isolation is accomplished by an immunoaffinity column. Immunoaffinity columns can take any form, but usually comprise a packed bed reactor. The packed bed in these bioreactors is preferably made of a porous material having a substantially uniform coating of a substrate. The porous material, which provides a high surface area-to-volume ratio, allows for the cell mixture to flow over a large contact area while not impeding the flow of cells out of the bed. Typical substrates include avidin and streptavidin, while other conventional substrates can be used. The substrate should, either by its own properties, or by the addition of a chemical moiety, display high-affinity for a moiety found on the cell binding protein such as a monoclonal antibody. The monoclonal antibodies recognize a cell surface antigen on the cells to be separated, and are typically further modified to present a biotin moiety. It is well-known that biotin has a high affinity for avidin, and the affinity of these substances thereby removably secures the monoclonal antibody to the surface of the packed bed. Such columns are well known in the art, see Berenson, et al., J. Cell Biochem, 10D:239 (1986). The column is washed with a PBS solution to remove unbound material. Target cells can be released from the beads using conventional methods. Immunoaffinity columns of the type described above that utilize biotinylated anti-CD34 monoclonal antibodies secured to an avidin-coated packed bed are described for example, in PCT Publ. No. WO 93/08268. A variation of this method utilizes cell binding proteins, such as the monoclonal antibodies or flt3-ligand as described above, removably-secured to a fixed surface in the isolating means. The bound cell binding protein then is contacted with the collected cell mixture and allowed to incubate for a period of time sufficient to permit isolation of the desired cells.

Alternatively, the monoclonal antibodies that recognize the cell surface antigens can be labeled with a fluorescent label, e,g., chromophore or fluorophore, and separated by cell sorting according to the presence of absence or the amount of labeled product.

The collected CD34⁺ cells are then exposed to either flt3-ligand alone or flt3-ligand in concurrent or sequential combination with one or more of the following cytokines: GM-CSF, TNF-α, IL-3, IL-4 c-kit-ligand or GM-CSF/IL-3 fusion proteins. CD34⁺ cells then are allowed to differentiate and commit to cells of the dendritic lineage. The dendritic cells are collected and can either be (a) administered to a patient in order to augment the immune system and Tell mediated or B-cell mediated immune responses to antigen, (b) exposed to an antigen prior to administration of the dendritic cells into a patient, (c) transfected with a gene encoding an antigen-specific polypeptide or (d) exposed to an antigen and then allowed to process and, resent the antigen, ex vivo, to T-cells collected from the patient followed by administration of the antigen-specific T-cells to the patient

More specifically, the invention provides for flt3-ligand for use as an adjuvant for increasing the number of dendritic cells. By increasing the quantity of the patient's dendritic cells, such cells may themselves be used to present antigen to T cells. For example, the antigen may be one that already exists within the patient, such as a tumor antigen, or a bacterial or viral antigen. Flt3-ligand may be used, therefore, to boost the patient's lymphocyte-mediated (e.g., T cell and B cell mediated) or myeloid-mediated immune response to the already present antigens thus potentially enabling a more effective antigen-presentation to the patient's T cells. Alternatively, flt3-ligand may be administered prior to, concurrently with or subsequent to administration of an antigen to a patient for immunization purposes. Thus, as a vaccine adjuvant, flt3-ligand can generate large quantities of dendritic cells in vivo to more effectively present the antigen. The overall response is a stronger and improved immune response and more effective immunization to the antigen.

The systemic administration of flt3-ligand not only is effective as a vaccine adjuvant, but as discussed supra., is effective in augmenting an immune response against previously existing antigens. For example, the inventors have shown that flt3-ligand administration to tumor-bearing mice results in at least a significant decrease in the growth rate of the tumor, and can result in tumor rejection in a large proportion of the mice. The data are presented in more detail in Example 3. Flt3-ligand therefore is an important cytokine in the generation of an effective immune response in vivo against antigen.

Because of its ability to generate dendritic cells, flt3-ligand also finds use in promoting the survival of transplanted tissue or organs. When allogeneic organs or other tissue is transplanted into a host they can transfer stem cells, immature dendritic cells, and mature dendritic cells from the donor. These cells are called passenger cells and such cells can graft into the hematopoietic system of the host. Additionally, stem cells, immature dendritic cells, and mature dendritic cells from the host may graft to the donor organ or tissue. It is possible then to establish a tolerance between the graft and the host since the immature dendritic cells from the host and donor tissue interact with T-cells from the "other side." Such interaction may include the deletion of T-cells that recognize the major histocompatability complex (MHC) that the dendritic cells express. In this way, the donor cells are "screened" so that they fail to recognize and react against the host (i.e.. no graft versus host disease) and the host T-cells are screened so that they fail to recognize and react against the graft (i.e., no graft rejection). Thus, a mutual tolerance can be achieved, and the graft acceptance is improved. Administration of flt3-ligand to the host or donor prior to transplantation would generate increased numbers of dendritic cells in such host or donor and permit increased tolerance and survival of the graft.

For the growth and culture of dendritic cells, a variety of growth and culture media can be used, and the composition of such media can be readily determined by a person having ordinary skill in the art. Suitable growth media are solutions containing nutrients or metabolic additives, and include those that are serum-depleted or serum-based. Representative examples of growth media are RPMI, TC 199, Iscoves modified Dulbecco's medium (Iscove, et al., F.J. Exp. Med., 147:923 (1978)), DMEM, Fischer's, alpha medium, NCTC, F-10, Leibovitz's L-15, MEM and McCoy's. Particular examples of nutrients that will be readily apparent to the skilled artisan include, serum albumin, transferrin, lipids, cholesterol, a reducing agent such as 2-mercaptoethanol or monothioglycerol, pyruvate, butyrate, and a glucocorticoid such as hydrocortisone 2-hemisuccinate. More particularly, the standard media includes an energy source, vitamins or other cell-supporting organic compounds, a buffer such as HEPES, Tris, that act to stabilize the pH of the media, various inorganic salts. Particular reference is made to PCT Publ. No. WO95/0063 wherein a variety of serum-free cellular growth media is described.

Peripheral blood progenitor cells (PBPC) and peripheral blood stem cells (PBSC) are collected using apheresis procedures known in the art. See, for example, Bishop et al., Blood, vol. 83, No. 2, pp. 610-616 (1994). Briefly, PBPC and PBSC are collected using conventional devices, for example, a Haemonetics Model V50 apheresis device (Haemonetics, Braintree, MA). Four-hour collections are performed typically no more than five times weekly until, for example, approximately 6.5 x 10⁸ mononuclear cells (MNC)/kg patient are collected. The cells are suspended in standard media and then centrifuged to remove red blood cells and neutrophils. Cells located at the interface between the two phases (also known in the art as the buffy coat) are withdrawn and resuspended in HBSS. The suspended cells are predominantly mononuclear and a substantial portion of the cell mixture are early stem cells. The resulting stem cell suspension then can be contacted with biotinylated anti-GD34 monoclonal antibodies or other cell-binding means. The contacting period is maintained for a sufficient time to allow substantial interaction between the anti-CD34 monoclonal antibodies and the CD34 antigens on the stem cell surface. Typically, times of at least one hour are sufficient. The cell suspension then is brought into contact with the isolating means provided in the kit. The isolating means can comprise a column packed with avidin-coated beads. Such columns are well known in the art, see Berenson, et al., J. Cell Biochem., 10D:239 (1986). The column is washed with a PBS solution to remove unbound material. Target stem cells can be released from the beads and from anti-CD34 monoclonal antibody using conventional methods. The stem cells obtained in this manner can be frozen in a controlled rate freezer (e.g., Cryo-Med, Mt. Clemens, MI), then stored in the vapor phase of liquid nitrogen. Ten percent dimethylsulfoxide can be used as a cryoprotectant. After all collections from the donor have been made, the stem cells are thawed and pooled. Aliquots containing stem cells, growth medium, such as McCoy's 5A medium, 0.3% agar, and at least one of the expansion factors: recombinant human GM-CSF, IL-3, recombinant human flt3-ligand, and recombinant human GM-CSF/IL-3 fusion molecules (PIXY321) at concentrations of approximately 200 U/mL, are cultured and expanded at 37°C in 5% CO₂ in fully humidified air for 14 days. Optionally, human IL-1α, or IL-4 may be added to the cultures. The most preferred combination of expansion factors comprises flt3-ligand plus either IL-3 or a GM-CSF/IL-3 fusion protein.

For in vivo administration to humans, flt3-ligand can be formulated according to known methods used to prepare pharmaceutically useful compositions. Flt3-liigand can be combined in admixture, either as the sole active material or with other known active materials, with pharmaceutically suitable diluents (e.g., Tris-HCl, acetate, phosphate), preservatives (e.g., Thimerosal, benzyl alcohol, parabens), emulsifiers, solubilizers, adjuvants and/or carriers. Suitable carriers and their formulations are described in Remington's Pharmaceutical Sciences, 16th ed. 1980, Mack Publishing Co. In addition, such compositions can contain flt3-ligand complexed with polyethylene glycol (PEG), metal ions, or incorporated into polymeric compounds such as polyacetic acid, polyglycolic acid, hydrogels, etc., or incorporated into liposomes, microemulsions, micelles, unilamellar or multilamellar vesicles, erythrocyte ghosts or spheroblasts. Such compositions will influence the physical state, solubility, stability, rate of *in vivo* release, and rate of *in vivo* clearance of flt3-ligand.

Flt3-ligand can be administered topically, parenterally, or by inhalation. The term "parenteral" includes subcutaneous injections, intravenous, intramuscular, intracisternal injection, or infusion techniques. These compositions will typically contain an effective amount of the flt3-ligand, alone or in combination with an effective amount of any other active material. Such dosages and desired drug concentrations contained in the compositions may vary depending upon many factors, including the intended use, patient's body weight and age, and route of administration. Preliminary doses can be determined according to animal tests, and the scaling of dosages for human administration can be performed according to art-accepted practices. Keeping the above description in mind, typical dosages of flt3-ligand may range from about 10 µg per square meter to about 1000 µg per square meter. A preferred dose range is on the order of about 100 µg per square meter to about 300 µg per square meter.

In addition to the above, the following examples are provided to illustrate particular embodiments and not to limit the scope of the invention.

### EXAMPLE 1

### Generation of Dendritic Cells

This Example describes a method for using flt3-ligand to generate large numbers of dendritic cells ex vivo. Cells having the CD34⁺ phenotype are isolated as described above, for example, first by generating a buffy coat of cells using a procedure described supra. Cells from the buffy coat are then incubated with a CD34 specific monoclonal antibody. The CD34⁺ cells which are selected then are cultured in McCoy's enhanced media with 20 ng/ml each of GM-CSF, IL-4, TNF-α, or 100 ng/ml flt3-ligand or c-kit ligand. The culture is continued for approximately two weeks at 37 °C in 10% CO₂ in humid air. Cells then are sorted by flow cytometry for CD1a⁺ and HLA-DR⁺ expression. The combination of GM-CSF, IL-4 and TNF-α, resulted in a six to seven-fold increase in the number of cells obtained after two weeks of culture. The combination of flt3-ligand and c-kit ligand resulted in an additive 12-13-fold increase in abolute cell numbers. This correlated with an 18-fold expansion with either flt3-ligand or c-kit ligand or to a 34-fold expansion with the combination of flt3-ligand and c-kit ligand. Phenotypic analysis of the cells showed that between 60-70% of the cells were HLA-DR⁺, CD86⁺, with 40-50% of the cells expressing CD1a in all factor combinations examined. The addition of flt3-ligand increased the absolute number of CD1a⁺ cells by 5-fold. c-Kit ligand increased those cells by 6.7-fold and the combination of flt3-ligand and c-kit ligand by 11-fold. Functional analysis of the resultant cells in an MLR revealed that the presence of flt3-ligand or c-kit ligand did not affect the stimulatory capacity of the resultant dendritic cells while increasing the numbers attained.

### EXAMPLE 2

### Use of Flt3-L in Dendritic Cell Expansion

This Example describes a method for using flt3-ligand for dendritic cell expansion. Prior to cell collection, it may be desirable to mobilize or increase the numbers of circulating PBPC and PBSC. Mobilization can improve PBPC and PBSC collection, and is achievable through the intravenous administration of flt3-ligand or sargramostim (Leukine^{®}, Immunex Corporation, Seattle, Washington) to the patients prior to collection of such cells. Other growth factors such as CSF-1, GM-CSF, c-kit ligand, G-CSF, EPO, IL-1, IL-2, IL-3, IL-4, IL-5, IL-6, IL-7, IL-8, IL-9, IL-10, IL-11, IL-12, IL-13, IL-14, IL-15, GM-CSF/IL-3 fusion proteins, LIF, FGF and combinations thereof, can be likewise administered in sequence, or in concurrent combination with flt3-ligand. Mobilized or non-mobilized PBPC and PBSC are collected using apheresis procedures known in the art. See, for example, Bishop et al., Blood, vol. 83, No. 2, pp. 610-616 (1994). Briefly, PBPC and PBSC are collected using conventional devices, for example, a Haemonetics Model V50 apheresis device (Haemonetics, Braintree, MA). Four-hour collections are performed typically no more than five times weekly until approximately 6.5 x 10⁸ mononuclear cells (MNC)/kg patient are collected. Aliquots of collected PBPC and PBSC are assayed for granulocyte-macrophage colony-forming unit (CFU-GM) content by diluting approximately 1:6 with Hank's balanced salt solution without calcium or magnesium (HBSS) and layering over lymphocyte separation medium (Organon Teknika, Durham, North Carolina). Following centrifugation, MNC at the interface are collected, washed and resuspended in HBSS. One milliliter aliquots containing approximately 300,000 MNC, modified McCoy's 5A medium, 0.3% agar, 200U/mL recombinant human GM-CSF, 200 u/mL recombinant human IL-3. and 200uEmL recombinant human G-CSF are cultured at 37°C in 5% CO₂ in fully humidified air for 14 days. Optionally, flt3-ligand or GM-CSF/IL-3 fusion molecules (PIXY 321) may be added to the cultures. These cultures are stained with Wright's stain, and CFU-GM colonies are scored using a dissecting microscope (Ward et al., Exp, Hematol., 16:358 (1988). Alternatively, CFU-GM colonies can be assayed using the CD34/C1733 flow cytometry method of Siena et al., Blood, Vol. 77, No. 2, pp 400-409 (1991), or any other method known in the art.

CFU-GM containing cultures are frozen in a controlled rate freezer (e.g., Cryo Med, Mt. Clemens, MI), then stored in the vapor phase of liquid nitrogen, Ten percent dimethylsulfoxide can be used as a cryoprotectant. After all collections from the patient have been made, CFU-GM containing cultures are thawed and pooled. The thawed cell collection is contacted with flt3-ligand either alone, sequentially or in concurrent combination with other cytokines listed above. Such exposure to flt3-ligand will drive the CFU-GM to dendritic cell lineage. The dendritic cells are reinfused intravenously to the patient.

### EXAMPLE 3

### Use of Flt3-1, in Augmenting Anti-tumor Immure Responses

This Example describes a method for using flt3-L to augment anti-tumor immune responses in vivo. Female C57BL/10J (B10) mice (The Jackson Laboratory, Bar Harbor, ME) were injected with 5 x 10⁵ viable B10.2 or B10.5 fibrosarcoma tumor cells by intradermal injection in a midline ventral position in a total volume of 50µl. The fibrosarcoma B10.2 andB10.5 lines are of B10 origin and have been described previously, see Lynch et al, Euro. J. Immunol. 21:1403 (1991). The fibrosarcoma B10.2 line was induced by subcutaneous implantation of a parrafin pellet containing 5 mg of methylcholanthrene, and the B10.5 line was induced by chronic exposure to ultraviolet radiation. The tumor cell lines were maintained in vitro in α-modified MEM containing 5% FBS, 2nM L-glutamine, 50U/ml penicillin and 50µg/ml streptomycin. Recombinant human flt3-L, (10µg/injection) was administered on a daily basis over a 19-day period (unless otherwise noted) by subcutaneous injection in a total volume of 100µl. Control mice were similarly injected with a similar volume of buffer containing 100 ng MSA. Tumor growth rates were determined by plotting the tumor size versus time after tumor challenge. Tumor size was calculated as the product of two perpendicular diameters, measured by calipers, and is expressed as the mean tumor size of only those mice bearing a tumor within a particular treatment group. The number of mice bearing tumors compared to the number challenged for each treatment group at the termination of an experiment are shown in the data below.

From Table I, the data is a compilation of six different experiments wherein tumor-bearing mice were either treated with flt3-ligand or MSA. Complete tumor regression was observed in 19 of 50 flt3-ligand treated mice compared to 1 of 30 in MSA-treated mice (p< 0.0001 using Fishers Exact Test). The observation that the rate of tumor growth in flt3-ligand treated mice (mean tumor size in tumor-bearing mice at week 5 post-tumor challenge was 60 +/- 8 mm²) was significantly reduced compared to MSA-treated mice (mean tumor size at week 5 post-tumor challenge was 185 +/- 17 mm²) was also confirmed (p.0001 by Analysis of Variance).

**TABLE I**

| Fibrosarcoma +/- Flt3-L Composite of Six Experiments Tumor Size (mm²) | | | | |
|---|---|---|---|---|
| Weeks Post Tumor Challenge | MSA Control (100ng/day) | Standard Error | Flt3-L (10µg/day) | Standard Error |
| 0 | 0 | 0 | 0 | 0 |
| 1 | 25 | 2.6 | 24 | 2.2 |
| 2 | 62 | 7.5 | 49 | 3.6 |
| 3 | 98 | 10.6 | 49 | 3.9 |
| 4 | 149 | 14.5 | 50 | 5 |
| 5 | 185 | 16.8 | 60 | 8.4 |

Tumor size was sharply retarded with flt3-ligand compared to the control. Therefore, the data show that flt3-ligand is an important cytokine in the augmentation of the immune response against foreign antigens, and in particular against cancer.

## Claims

1. Flt3-ligand for use as a vaccine adjuvant for increasing the number of dendritic cells.

2. Use of a flt3-ligand in the manufacture of a vaccine adjuvant for increasing the number of dendritic cells.

3. The flt3-ligand according to claim 1, wherein the flt3-ligand is for use prior to, concurrently with or subsequent to administration of a vaccine antigen.

4. The flt3-ligand according to claim 1 or claim 3 in combination with any one of GM-CSF, IL-4, TNF-α, IL-3, c-kit ligand and fusions of GM-CSF and IL-3.

5. Flt3-ligand for use in increasing dendritic cells in a host, a donor or both undergoing a transplantation procedure, whereby the increased number of dendritic cells inhibit graft versus host and host versus graft disease.

6. The use of a flt3-ligand in the manufacture of a medicament for increasing dendritic cells in a host, a donor or both undergoing a transplantation procedure, whereby the increased number of dendritic cells inhibit graft versus host and host versus graft disease.

7. Use of flt3-ligand and any one of cytokines IL-3, IL-4, GM-CSF, TNF, C-Kit ligand and GM-CSF/IL-3 fusion proteins in a cell expansion media for generating dendritic cells *in vitro.*

8. Flt3-ligand for use in treating fibrosarcoma.

9. Use of a flt3-ligand in the manufacture of a medicament for treating fibrosarcoma.

## Patentansprüche

1. Flt3-Ligand für die Verwendung als Vakzin-Adjuvans zur Erhöhung der Anzahl an dentritischen Zellen.

2. Verwendung eines Flt3-Liganden bei der Herstellung eines Vakzin-Adjuvans zur Erhöhung der Anzahl an dentritischen Zellen.

3. Flt3-Ligand nach Anspruch 1, wobei der Flt3-Ligand zur Verwendung vor, gleichzeitig mit oder nach Verabreichung eines Vakzin-Antigens vorgesehen ist.

4. Flt3-Ligand nach Anspruch 1 oder 3 in Kombination mit einem von GM-CSF, IL-4, TNF-α, IL-3, c-Kit-Ligand und Fusionen von GM-CSF und IL-3.

5. Flt3-Ligand für die Verwendung bei der Erhöhung von dentritischen Zellen in einem Wirten, einem Spender oder sowohl als auch, bei dem ein Transplantationsvorgang durchgeführt wird, wobei die erhöhte Anzahl an dentritischen Zellen die Transplantat-gegen-Wirt-Erkrankung und die Wirt-gegen-Transplantat-Erkrankung inhibiert.

6. Verwendung eines Flt3-Liganden bei der Herstellung eines Medikaments zur Erhöhung von dentritischen Zellen in einem Wirten, einem Spender oder sowohl als auch, bei dem ein Transplantationsvorgang durchgeführt wird, wobei die erhöhte Anzahl an dentritischen Zellen die Transplantat-gegen-Wirt-Erkrankung und die Wirt-gegen-Transplantat-Erkrankung inhibiert.

7. Verwendung eines Flt3-Liganden und eines von den Cytokinen IL-3, IL-4, GM-CSF, TNF, c-Kit-Ligand und GM-CSF/IL-3-Fusionsproteinen in einem Zellexpansionsmedium zum Erzeugen von dentritischen Zellen in vitro.

8. Flt3-Ligand für die Verwendung bei der Fibrosarkom-Behandlung.

9. Verwendung eines Flt3-Liganden bei der Herstellung eines Medikaments zur Fibrosarkom-Behandlung.

## Revendications

1. Ligand-flt3 à utiliser comme adjuvant à un vaccin pour augmenter le nombre de cellules dendritiques.

2. Utilisation d'un ligand-flt3, dans la fabrication d'un adjuvant à un vaccin pour augmenter le nombre de cellules dendritiques.

3. Ligand flt-3 selon la revendication 1, où le ligand flt-3 est destiné à une utilisation avant, pendant ou après l'administration d'un antigène de vaccin.

4. Ligand flt-3 selon la revendication 1 ou la revendication 3, en combinaison avec l'un quelconque des éléments parmi GM-VSF, IL-4, TNF-α, IL-3, ligand de c-kit et fusions de GM-CSF et IL-3.

5. Ligand flt-3 à utiliser dans l'augmentation de cellules dendritiques dans un hôte, un donneur ou les deux subissant une procédure de transplantation, moyennant quoi le nombre accru de cellules dendritiques inhibe une réaction du greffon contre l'hôte et de l'hôte contre le greffon.

6. Utilisation d'un ligand flt-3 dans la fabrication d'un médicament pour augmenter les cellules dendritiques sur un hôte, un donneur ou les deux, subissant une procédure de transplantation, moyennant quoi le nombre accru de cellules dendritiques inhibe la réaction du greffon contre l'hôte et de l'hôte contre le greffon.

7. Utilisation d'un ligand flt-3 et de l'une quelconque des cytokines IL-3, IL-4, GM-CSF, TNF, ligand de c-kit et des protéines de fusion GM-CSF/IL-3 dans un milieu d'expansion cellulaire pour générer des cellules dendritiques in vitro.

8. Ligand flt-3 à utiliser dans le traitement du fibrosarcome.

9. Utilisation d'un ligand flt3 dans la fabrication d'un médicament pour traiter le fibrosarcome.
